# EUROPEAN PATENT SPECIFICATION

(11) **EP 0 510 185 B1**
(45) Date of publication and mention of the grant of the patent: **11.12.1996**
(21) Application number: 92902017.0
(22) Date of filing: 30.10.1991
(51) Int. Cl.: A01N 1/02

(54) **BLOOD PLATELET STORAGE MEDIUM**
MEDIUM ZUR AUFBEWAHRUNG VON BLUTPLÄTTCHEN
MILIEU DE STOCKAGE DE PLAQUETTES DE SANG

(30) Priority: 07.11.1990 US 610488
(43) Date of publication of application: 28.10.1992
(73) Proprietor: BAXTER INTERNATIONAL INC., Deerfield, Illinois 60015 (US)
(72) Inventor: GRODE, Gerald, A., Lake Villa, IL 60064 (US); BISCHOF, Daniel, McHenry, IL 60050 (US); GOLDHABER, Richard, Lake Forest, IL 60045 (US)
(74) Representative: MacGregor, Gordon
(86) International application number: US9108116
(87) International publication number: WO9208349

(56) References cited:
- EP-A- 76 658
- EP-A- 0 099 315
- EP-A- 0 301 250
- EP-A- 0 313 808
- WO-A-86/00809
- DD-A- 152 719
- US-A- 32 874
- US-A- 3 874 384
- US-A- 4 267 269
- US-A- 4 704 352
- CHEMICAL ABSTRACTS, vol. 89, no. 19, 1978, Columbus, Ohio, US; abstract no. 160846w, MEZEY ET AL. 'Study and use of the acd-adenine-guanosine blood stabiliser'
- CHEMICAL ABSTRACTS, vol. 87, no. 13, 1977, Columbus, Ohio, US; abstract no. 99582x, MESSETER ET AL. 'cpd-adenine as a blood preservative-studies in vitro and in vivo'
- Week 8922, Derwent Publications Ltd., London, GB; AN 89-162877
- Vox Sanguinis, Volume 47, issued 1984, K. KOERNER, "Platelet function of room temperature platelet concentrates stored in a new plastic material with high gas permeability", pages 406-411, see Abstract.

## Description

### BACKGROUND OF THE INVENTION

The present invention relates generally to the storage of blood components. More specifically, the present invention relates to the storage of blood components, such as red blood cells and platelets.

Blood comprises two principal parts. When blood is collected and centrifuged, the blood separates into: plasma; and packed red cells.

Plasma is comprised of water and inorganic and organic substances as well as dissolved gases and miscellaneous foreign components. The inorganic substances contained in plasma are primarily electrolytes. The electrolytes include: sodium; potassium; calcium; magnesium; chloride; bicarbonate; phosphate; and sulfate. The most significant organic substances found in plasma are: lactic acid; urea; amino acids; creatinine; glucose; hormones; proteins; albumins; and globulins.

In certain disease states and traumas, it is necessary to infuse into a patient red blood cells and/or platelets. For example, if the platelet count in the circulatory system drops below a lower limit of approximately 70,000 per cubic millimeters, thrombocytopenia can result. Thrombocytopenia results in a tendency to bleed, similar to hemophiliacs, except that bleeding is typically from many small capillaries rather than from large vessels as in hemophilia. A person suffering from thrombocytopenia typically suffers small hemorrhages throughout their body tissue. The infusion of platelets is particularly important to repair the minute breaks in capillaries and other small vessels.

Platelets can be obtained as a by-product from whole blood donations and from plateletpheresis. Typically, platelets are stored in their own plasma within a plastic container. Usually, the plasma contains all of the ingredients found in physiological plasma and an anticoagulant, typically a citrate. Additionally, dextrose is present at an amount greater than the physiological level, for example five (5) times the physiological level, for the benefit of red cells that require it during storage and is generally accepted to be required for platelet storage as well.

There are known methods for storing platelets and red blood cells. In one type of method, the platelets are suspended in plasma; there are several disadvantages to storing platelets or red blood cells in large volumes of plasma, and then infusing the platelets or red blood cells into a recipient.

One of the principal disadvantages is the transmission of diseases due to the infusion of plasma. Certain disease states including hepatitis-B and nonA, nonB - hepatitis and acquired immune deficiency syndrome (AIDS) can be transmitted through plasma infusion. Furthermore, patients can also exhibit allergic reactions to plasma. Additionally, plasma is valuable because it can be fractioned into its various components such as albumin and coagulation factors for treatment of specific disease states. The use of plasma as a storage medium for platelets or red blood cells wastes valuable plasma.

There have been many attempts at creating a plasma free storage medium for platelets and red blood cells. Examples of such attempts at creating platelet storage medium are set forth in U.S. Patent Nos. 4,695,460, Re. 32,874, and European Patent Application No. EP 0 142 339. Additionally, various preservatives in culture medium are described as being useful in conjunction with the storage of platelets. EPO 142 339 notes that examples of such preservatives are set forth in U.S. Patent Nos.: 2,786,014; 3,629,071; 3,753,357; 3,814,687; 3,850,174; 4,152,208; 4,205,126; 4,267,269; and 4,390,619; as well as GB-A-1,283,273.

Blood cell component storage solutions are also disclosed in U.S. Patent Nos.: 4,704,352; 4,675,185; 4,585,735; and EP 0 044 864.

In order to store human blood components such as red blood cells and platelets, the storage medium must provide cell nutrients, simple sugars, such as glucose. Glucose is known to degrade under autoclaving (heat) sterilization conditions unless the medium being heated is acidic, i.e., has a pH of less than 7. If the solution is not acidic, when heated, glucose will caramelize.

In order to remain viable, a cell must generate ATP (adenosine triphosphate) to meet its requisite energy requirements. ATP can be generated through two pathways, glycolysis and oxidative phosphorylation. Cell glycolysis (glycolytic pathway) results in the production of quantities of lactic acid (one mole of glucose is converted to two moles of lactic acid to achieve two molecules of ATP). Lactic acid (having a pKa = 3) is immediately neutralized by plasma bicarbonate buffer (pKa = 6.3) to produce sodium lactate and carbonic acid (H₂CO₃). Carbonic acid is at equilibrium with carbon dioxide and water (H₂CO₃ ⇄ CO₂ + H₂O).

During blood component storage, especially platelet storage, the carbonic acid (H₂CO₃) is removed by the diffusion of CO₂ through a permeable container. A buffer system therefore offers a potential for acid removal via gas diffusion. However, the buffer system is not stable under low pH conditions due to the high level of H₂CO₃ and associated high CO₂ tensions unless it is packaged in an impermeable container, such as glass.

Furthermore, in addition to production and storage problems of a buffered nutrient blood component storage medium, it must also be noted that the medium for storing plasma depleted cells, such as platelets and red blood cells, must be at a proper physiological pH.

EP-A-313808 discloses a blood component (e.g. red blood cell) storage medium including dextrose and bicarbonate at a pH of 6.8 to 7.4. This document discloses that the dextrose can be separately sterilized from the bicarbonate, because dextrose is not readily sterilized above pH 6.4.

Recently, white blood cells have been collected as a separate component for a number of reasons, e.g. gene transfer therapy. The white blood cells are preserved and/or manipulated for such purposes. Typically, the white blood cells have been stored in complex storage solutions.

EP-A-76658 discloses an ophthalmic irrigation solution (pH 6.8 to 8.0) made up immediately before use by combining a first, bicarbonate solution (pH 7.2 to 7.8) with a second, dextrose solution (pH < 5). The first and second solutions are autoclaved and stored separately.

The present invention provides a blood component storage apparatus as set out in Claim 1.

The precharacterising part of Claim 1 is based on EP-A-313808, and the distinguishing features of the present invention are set out in the characterising part of Claim 1.

Additional features and advantages of the present invention are described in, and will be apparent from, the detailed description of the presently preferred embodiments and from the drawings.

### BRIEF DESCRIPTION OF THE DRAWINGS

Figure 1 illustrates a perspective view of a first and second container including the first and second solutions and a third container including a plasma depleted blood component.

Figure 2 illustrates a perspective view of the first and second containers after the redistribution of the storage medium and resuspended plasma depleted blood component to the first and second containers.

### DETAILED DESCRIPTION OF THE PRESENTLY PREFERRED EMBODIMENTS

A blood component storage medium is provided that has improved shelf storage properties and comprises two distinct solutions. By providing two distinct solutions, improved manufacturing and storage properties are achieved. The two solutions, when stored separately, have a shelf life of at least approximately three (3) years.

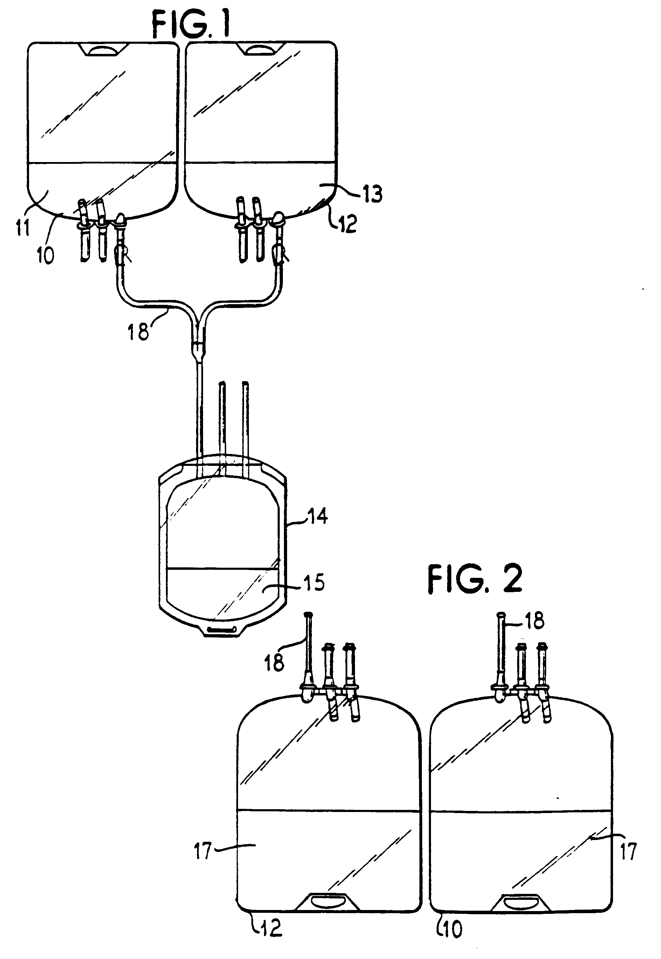

As illustrated in Figure 1, a first and second container, 10 and 12, respectively, are provided. The containers 10 and 12 are constructed from a gas permeable plastic material, such as, for example, ethylenevinyl acetate. The permeability of the plastic containers 10 and 12 allows CO₂ to diffuse through the container removing carbonic acid (H₂CO₃) from the solution housed in the container. This is especially critical when the blood component is suspended in the storage medium.

The first container 10 includes a first solution 11 including a nutrient source for the blood component. Although, preferably, the nutrient source is a simple sugar, any sugar that can be metabolized will provide the nutrient source. In an embodiment, the nutrient source is dextrose (glucose). The dextrose provides an energy source for the cells of the blood component and allows the cells to generate the necessary ATP to meet the requisite energy requirements of the blood component.

The first solution 11 also preferably includes necessary electrolytes and anticoagulants and other components necessary for the storage of the blood components. For example, in an embodiment, the first solution includes: citric acid; sodium citrate; dextrose; adenine; sodium chloride; potassium chloride; calcium chloride; and magnesium chloride.

Due to its components, the first solution 11 must be maintained, during its sterilization and storage, in an acidic condition. To this end, the first solution is maintained at a pH of less than 7, preferably, the pH of the solution is less than 6. It has been found that the first solution 11 should have a pH of approximately 5 to about 6.

The second solution 13, that acts as a buffer, is also housed in a gas permeable container 12. In an embodiment, the second solution preferably includes a bicarbonate buffer. In an embodiment, the second solution 13 includes sodium phosphate and sodium bicarbonate.

The second solution 13 is maintained at a basic pH and accordingly has a pH of greater than 7. Preferably, the pH of the buffer is greater than 8. It has been found that a pH of approximately 8 to about 9 functions satisfactorily.

In use, the first and second solutions are stored separately and mixed together to create the blood component storage medium. The storage medium is added to the plasma depleted blood cell component.

To this end, the first solution 11 is drained into the second container 12, or vice versa, utilizing a tubing 18 that prevents communication between the containers 10 and 12. As is known in the art, frangible members are located in the tubing 18 that can be fractured to establish fluid flow between the first container 10 and second container 12, and ultimately a third container 14.

The resultant solution, from the mixing of the first and second solutions, is then added to a third container 14 that includes the plasma depleted blood cell component 15. The third container 14 can contain, for example, a platelet, a white blood cell, or red blood cell button. The blood cell components 15 are then resuspended in the resultant solution by gentle agitation of the third container 14. The solution 17 containing the blood cell components is then redistributed into the first and second containers 10 and 12, respectively.

As illustrated in Figure 2, after the solution 17 is redistributed, the first and second containers 10 and 12 are then sealed, by cutting and sealing the tubing 18, creating two containers of solution and blood cell component, for example, platelets in a storage medium. Because the first and second containers are gas permeable, they can be used to house the blood cell component and storage medium.

The first and second solutions are constructed so that when they are combined they have a physiologically acceptable pH. To this end, the two solutions when combined have a pH of approximately 6.8 to about 7.4. Most preferably, the two solutions when combined have a pH of approximately 7.0 to about 7.2.

The present invention also provides a method for storing blood components. The method comprises the steps of providing a first container 10 including a first solution 11 having a sugar nutrient and a pH of less than 7. A second container 12 is provided including a buffer second solution 13 and having a pH greater than 7. The first and second solutions 11 and 13 are mixed together to create a storage medium. The storage medium is added to a plasma depleted collection of blood components 15, such as platelets. The blood component and storage medium can then be redistributed to the containers that are then sealed. The containers can then house the blood component until it is infused into a patient.

In an embodiment, the solution can be used to store white blood cells. White blood cells can be collected for a number of purposes. For example, the white blood cells can be collected and modified for gene transfer therapy. To this end, a gene can be inserted into the white blood cells using a retrovirsus.

By way of example, and not limitation, an example of the present invention is set forth below.

A first 1000 ml container available from Baxter Healthcare International under the designation P1 732 plastic is provided that includes a first solution A. A second 1000 ml container, that is the same type of container as the first container, is provided and includes a second solution B. The solutions A and B are as follows:

| SOLUTION A | | | |
|---|---|---|---|
| COMPONENT | HYDRATE | mg/dL | mM |
| CITRIC ACID | Mono | 112 | 0.5 |
| SODIUM CITRATE | Di | 895 | 3.0 |
| DEXTROSE | Mono | 1980 | 10.0 |
| ADENINE | - | 60 | 0.4 |
| SODIUM CHLORIDE | - | 1290 | 22.1 |
| POTASSIUM CHLORIDE | - | 76 | 1.0 |
| CALCIUM CHLORIDE | Di | 50 | 0.3 |
| MAGNESIUM CHLORIDE | Hexa | 32.5 | 0.2 |

| SOLUTION B | | | |
|---|---|---|---|
| SODIUM PHOSPHATE | - | 85 | 0.6 |
| SODIUM BICARBONATE | - | 504 | 6.0 |

100 milliliters of solution A is present in the first container and 100 milliliters of solution B is present in the second container.

Plasma depleted platelets are obtained from whole blood and housed in a container, for example, a container available from Baxter Healthcare under the designation P1 732 plastic, as a platelet button. The platelet button can be obtained by plateletpheresis of the whole blood. Approximately 3 x 10¹¹ to about 6 x 10¹¹ platelet cells are present in the button.

The first and second solutions are mixed together and then added to the third container. The platelets are then resuspended in the solution and the platelets and solution are distributed to the first and second containers creating two containers housing 100 ml of platelets in storage medium. The platelets can be stored in the medium for approximately five (5) days.

## Claims

1. A method for storing blood components comprising providing a first, carbon dioxide-permeable storage container including a first solution having a sugar nutrient and a pH of less than 7;
providing a second, carbon dioxide-permeable storage container including a buffer second solution and a pH of greater than 7;
mixing the first and second solutions together to create a storage medium;
adding a blood component to the storage medium and
storing first and second amounts of the blood component and storage medium mixture in a respective one of said containers (10,12).

2. The method of Claim 1 including the step of autoclaving the first and second containers (10,12) including the solutions to sterilize the first and second containers prior to mixing the solutions to create the storage medium.

3. The method of Claim 1 or 2 wherein the blood component is plasma depleted platelets, plasma depleted red blood cells, or plasma depleted white blood cells.

4. A blood component storage apparatus for use in the method of Claim 1, the apparatus comprising:
a first container (10) containing a solution including at least one sugar nutrient and a pH of less than 7; and
a second container (12) containing a second solution including a bicarbonate buffer and having a pH of greater than 7, the two solutions being mixed together prior to use to form a storage medium;
characterised in that each container (10,12) is a carbon dioxide-permeable solution storage container.

5. The apparatus of Claim 4 wherein the first solution includes dextrose.

6. The apparatus of Claim 4 wherein the first solution includes: citric acid; sodium citrate; dextrose; adenine; sodium chloride; potassium chloride; calcium chloride; and magnesium chloride.

7. The apparatus of Claim 4, 5 or 6 wherein the second solution includes sodium phosphate and sodium bicarbonate.

8. The apparatus of any one of Claims 4 to 7, wherein the pH of the first solution is less than 6 and preferably between 5 and 6.

9. The apparatus of any one of Claims 4 to 8, wherein the pH of the second solution is at least 8 and preferably between 8 and 9.

10. The apparatus of any one of Claims 4 to 9, wherein when mixed together, the solutions form a storage medium having a pH of 6.8 to 7.4.

## Patentansprüche

1. Verfahren zur Lagerung von Blutbestandteilen, welches folgende Schritte aufweist:
- es wird ein erster, für Kohlendioxid durchlässiger Lagerbehälter mit einer ersten Lösung bereitgestellt, die einen Zuckernährstoff enthält und einen pH-Wert unter 7 aufweist;
- es wird ein zweiter, für Kohlendioxid durchlässiger Lagerbehälter bereitgestellt, der als zweite Lösung eine Pufferlösung enthält und einen pH-Wert über 7 aufweist;
- die erste und die zweite Lösung werden zur Bildung eines Lagermediums miteinander vermischt;
- dem Lagermedium wird ein Blutbestandteil zugefügt; und
- eine erste und eine zweite Menge des Blutbestandteils und des das Lagermedium bildenden Gemisches werden jeweils in einem der Behälter (10, 12) gelagert.

2. Verfahren nach Anspruch 1,
welches den Schritt umfaßt, daß der erste und der zweite Behälter (10, 12), welche die Lösungen enthalten, in einem Autoklaven behandelt werden, um den ersten und den zweiten Behälter vor dem Mischen der Lösungen zu sterilisieren, um das Lagermedium zu erzeugen.

3. Verfahren nach Anspruch 1 oder 2,
wobei der Blutbestandteil aus plasmaverarmten Blutplättchen, plasmaverarmten roten Blutkörperchen oder plasmaverarmten weißen Blutkörperchen besteht.

4. Vorrichtung zur Lagerung von Blutkomponenten zur Verwendung bei dem Verfahren gemäß Anspruch 1, wobei die Vorrichtung folgendes aufweist:
- einen ersten Behälter (10), der eine Lösung enthält, die mindestens einen Zuckernährstoff enthält und einen pH-Wert von weniger als 7 aufweist; und
- einen zweiten Behälter (12), der eine zweite Lösung enthält, die einen Bikarbonatpuffer enthält und einen pH-Wert von mehr als 7 aufweist, wobei die beiden Lösungen vor der Verwendung miteinander vermischt werden, um ein Lagermedium zu bilden,
**dadurch gekennzeichnet,**
daß jeder Behälter (10, 12) ein für Kohlendioxid durchlässiger Lagerbehälter zur Lagerung von Lösungen ist.

5. Vorrichtung nach Anspruch 4,
wobei die erste Lösung Dextrose enthält.

6. Vorrichtung nach Anspruch 4,
wobei die erste Lösung folgendes enthält: Zitronensäure; Natriumcitrat; Dextrose; Adenin; Natriumchlorid; Kaliumchlorid; Kalziumchlorid; und Magnesiumchlorid.

7. Vorrichtung nach Anspruch 4, 5 oder 6,
wobei die zweite Lösung Natriumphosphat und Natriumbicarbonat enthält.

8. Vorrichtung nach einem der Ansprüche 4 bis 7,
wobei der pH-Wert der ersten Lösung weniger als 6 beträgt und vorzugsweise zwischen 5 und 6 liegt.

9. Vorrichtung nach einem der Ansprüch 4 bis 8,
wobei der pH-Wert der zweiten Lösung mindestens 8 beträgt und vorzugsweise zwischen 8 und 9 liegt.

10. Vorrichtung nach einem der Ansprüche 4 bis 9,
wobei die Lösungen, wenn sie miteinander vermischt sind, ein Lagermedium mit einem pH-Wert von 6,8 bis 7,4 bilden.

## Revendications

1. Procédé pour conserver des composants sanguins, qui consiste à disposer un premier récipient de conservation, perméable au dioxyde de carbone, comprenant une première solution ayant un nutriment de type sucre et un pH inférieur à 7 ;
disposer un deuxième récipient de conservation, perméable au dioxyde de carbone, comprenant une deuxième solution tampon à un pH supérieur à 7 ;
mélanger ensemble les première et deuxième solutions pour créer un milieu de conservation ;
ajouter un composant sanguin au milieu de conservation ; et
conserver des premières et deuxième quantités du mélange de composant sanguin et de milieu de conservation dans un récipient respectif parmi lesdits récipients (10, 12).

2. Procédé selon la revendication 1, comprenant l'étape consistant à soumettre à un traitement à l'autoclave les premier et deuxième récipients (10, 12) comprenant les solutions, pour stériliser les premier et deuxième récipients avant de mélanger les solutions pour créer le milieu de conservation.

3. Procédé selon la revendication 1 ou 2, dans lequel le composant sanguin est constitué par des plaquettes appauvries en plasma, des globules rouges appauvris en plasma ou des globules blancs appauvris en plasma.

4. Appareil de conservation de composants sanguins destiné à être utilisé dans le procédé selon la revendication 1, l'appareil comprenant :
un premier récipient (10) contenant une solution comprenant au moins un nutriment de type sucre et ayant un pH inférieur à 7 ; et
un deuxième récipient (12) contenant une deuxième solution comprenant un tampon bicarbonate et ayant un pH supérieur à 7, les deux solutions étant mélangées ensemble avant d'être utilisées pour former un milieu de conservation ;
caractérisé en ce que chaque récipient (10, 12) est un récipient de conservation de solution perméable au dioxyde de carbone.

5. Appareil selon la revendication 4, dans lequel la première solution comprend du dextrose.

6. Appareil selon la revendication 4, dans lequel la première solution comprend de l'acide citrique, du citrate de sodium, du dextrose, de l'adénine, du chlorure de sodium, du chlorure de potassium, du chlorure de calcium et du chlorure de magnésium.

7. Appareil selon la revendication 4, 5 ou 6, dans lequel la deuxième solution comprend du phosphate de sodium et du bicarbonate de sodium.

8. Appareil selon l'une quelconque des revendications 4 à 7, dans lequel le pH de la première solution est inférieur à 6 et est de préférence compris entre 5 et 6.

9. Appareil selon l'une quelconque des revendications 4 à 8, dans lequel le pH de la deuxième solution est d'au moins 8 et est de préférence compris enter 8 et 9.

10. Appareil selon l'une quelconque des revendications 4 à 9, dans lequel, lorsqu'elles sont mélangées ensemble, les solutions forment un milieu de conservation ayant un pH compris entre 6,8 et 7,4.
